# EUROPEAN PATENT SPECIFICATION

(11) **EP 1 435 943 B1**
(45) Date of publication and mention of the grant of the patent: **14.02.2007**
(21) Application number: 02774687.4
(22) Date of filing: 07.10.2002
(51) Int. Cl.: A61K 31/4015, A61P 25/14

(54) **USE OF 2-OXO-1-PYRROLIDINE DERIVATIVES FOR THE PREPARATION OF A DRUG FOR TREATING DYSKINESIA**
VERWENDUNG VON 2-OXO-1-PYRROLIDIN-DERIVATEN ZUR HERSTELLUNG EINES ARZNEIMITTELS ZUR BEHANDLUNG VON DYSKINESIA
UTILISATION DE DERIVES DE 2-OXO-1-PYRROLIDINE POUR LA PREPARATION D'UN MEDICAMENT POUR TRAITER LA DYSKINESIE

(30) Priority: 08.10.2001 EP 01123976
(43) Date of publication of application: 14.07.2004
(73) Proprietor: UCB, S.A., 1070 Bruxelles (BE)
(72) Inventor: GRIMEE, Renee, B-1180 BRUSSELS (BE); KLITGAARD, Henrik, B-1180 BRUSSELS (BE)
(74) Representative: UCB Intellectual Property
(86) International application number: PCT/EP2002/011203
(87) International publication number: WO 2003/030899

(56) References cited:
- WO-A-01/39779
- WO-A-01/62726
- GB-A- 1 309 692
- US-A- 4 696 943
- ROSSI F. ET AL: "[Nootropic drugs]. I FARMACI NOOTROPI." RIFORMA MEDICA, (1993) 108/1 (1-14)., XP008019117
- GRANT R.; SHORVON S.D.: 'Efficacy and tolerability of 1000-4000 mg per day of levetiracetam as add-on therapy in patients with refractory epilepsy. AU PUB Netherlands' EPILEPSY RESEARCH vol. 42, no. 2-3, 2000, pages 89 - 95
- RIGO J.-M. ET AL: 'The anti-epileptic drug levetiracetam reverses the inhibition by negative allosteric modulators of neuronal GABA- and glycine-gated currents' BRITISH JOURNAL OF PHARMACOLOGY JULY vol. 136, no. 5, 2002, pages 659 - 672, XP008049203

## Description

The present invention relates to the use of 2-oxo-1-pyrrolidine derivatives and in particular (S)-(-)-α-ethyl-2-oxo-1-pyrrolidineacetamide for the preparation of drugs for the curative and/or prophylactic treatment of movement disorders or dyskinesia.

Movement and other disorders due to dysfunction of the basal ganglia and related brain structures are of major socio-economic importance. Such disorders can occur as a consequence of inherited or acquired disease, idiopathic neurodegeneration or they may be iatrogenic. The spectrum of disorders is very diverse, ranging from those associated with poverty of movement (akinesia, hypokinesia, bradykinesia) and hypertonia (e.g. Parkinson's disease) to the involuntary movement disorders (hyperkinesias or dyskinesias e.g. Huntington's disease, L-DOPA-induced dyskinesia, tardive dyskinesia, progressive supernuclear palsy , multiple system atrophy, corticobasal degeneration, Wilson's disease, progressive pallidal atrophy).

Parkinson's disease and related conditions represent one of the most prevalent diseases associated with poverty of movement. Parkinsonian symptoms manifest as a syndrome of symptoms characterised by slowness of movement (bradykinesia), rigidity and /or tremor. Parkinsonian symptoms are seen in a variety of conditions, most commonly in idiopathic parkinsonism (i.e. Parkinson's Disease) but also following treatment of schizophrenia (i.e. neuroleptic induced parkinsonism), exposure to toxins/drugs and head injury.

It is widely appreciated that the primary pathology underlying Parkinson's disease is degeneration, in the brain, of the dopaminergic projection from the substantia nigra to the striatum. This has led to the widespread use of dopamine-replacing agents (e.g. L-3,4-dihydroxyphenylalanine (L-DOPA) and dopamine agonists) as symptomatic treatments for Parkinson's disease. Such treatments have been successful in increasing the quality of life of patients suffering from Parkinson's disease. However, dopamine-replacement treatments do have limitations, especially following long-term treatment. Problems can include fluctuations (e.g. "on-off "phenomenon, wearing-off of the anti-parkinsonian efficacy of the treatment) and the appearance of a range of side-effects which manifest as abnormal involuntary movements, such as dyskinesias.

Dyskinesias, as a whole, are characterised by the development in a subject of abnormal involuntary movements. One way in which dyskinesias may arise is as a side effect of dopamine replacement therapy for parkinsonism or other basal ganglia-related movement disorders.

Many attempts have been made to identify agents that will prevent the development of, and/or treat dyskinesias although such attempts have met with limited success. There is therefore, a need to discover ways by which movement disorders and dyskinesias may be treated.

The use of levorotatory (S)-α-ethyl-2-oxo-1-pyrrolidineacetamide, also known as levetiracetam [International Nonproprietary Name] as a protective agent for the treatment and prevention of hypoxic and ischaemic type aggressions of the central nervous system is described in the European patent EP-A-0162 036. That compound can also be employed in the treatment of epilepsy, a therapeutic indication for which it has been demonstrated that its dextrorotatory enantiomer, (R)-(+)-α-ethyl-2-oxo-1-pyrrolidine-acetamide, is completely devoid of activity (A. J. GOWER et al., Eur. J. Pharmacol., 222, (1992). 193-203). That compound has also been described in European patent EP-A-0 645 139 for the treatment of anxiety.

EP-A-162 036 cited above also describes methods for preparing (S)-(-)-α-ethyl-2-oxo-1-pyrrolidine-acetamide which require the synthesis of a starting reactant obtained by resolution of the corresponding racemate. British patent GB-A-2 225 322 describes a method for preparing that compound which offers the advantage of using a natural amino acid which already has the desired stereochemical configuration as the starting material, thus dispensing with any laborious separation of the enantiomers.

2-oxo-1-pyrrolidine derivatives are described in the international patent application WO O1/62726 as well as their use as pharmaceuticals. The derivatives are particularly suited for treating neurological disorders such as epilepsy.

In continuing its research on these compounds, the Applicant has now discovered that (S)-(-)-α-ethyl-2-oxo-1-pyrrolidineacetamide and also 2-oxo-1-pyrrolidine derivatives possess therapeutic properties which render it particularly useful in the treatment and prophylaxis of movement disorders and dyskinesia.

The present invention thus concerns the use of an active compound which is a 2-oxo-1-pyrrolidine derivative selected from (S)-(-)-α-ethyl-2-oxo-1-pyrrolidine acelamide and ((2S)-2-[(4S)-4-(2,2-difluoro-vinyl)-2-oxopyrrolidinyl] butanamide or a pharmaceutically acceptable salt thereof for the manufacture of a medicament for the treatment of Parkinson's Disease in a dopamine replacement therapy using L- DOPA. or a pharmaceutically acceptable salt thereof.

Pharmaceutical compositions comprising the active compound can, for example, be administered orally or parenterally, i.e., intravenously, intramuscularly or subcutaneously, intrathecally.

Pharmaceutical compositions which can be used for oral administration can be solids or liquids and can, for example, be in the form of tablets, pills, dragees, gelatin capsules, solutions, syrups, and the like.

To this end, the active compound can be used mixed with an inert diluent or a nontoxic pharmaceutically acceptable vehicle such as starch or lactose, for example. Optionally, these pharmaceutical compositions can also contain a binder such as microcrystalline cellulose, gum tragacanth or gelatine, a disintegrant such as alginic acid, a lubricant such as magnesium stearate, a glidant such as colloidal silicon dioxide, a sweetener such as sucrose or saccharin, or colouring agents or a flavouring agent such as peppermint or methyl salicylate. They also comprise compositions which can release the active substance in a controlled manner. Pharmaceutical compositions which can be used for parenteral administration are in the pharmaceutical forms which are known for this mode of administration and are in the form of aqueous or oily solutions or suspensions generally contained in ampoules, disposable syringes, glass or plastics vials or infusion containers.

In addition to the active compound, these solutions or suspensions can optionally also contain a sterile diluent such as water for injection, a physiologic saline solution, oils, polyethylene glycols, glycerine, propylene glycol or other synthetic solvents, antibacterial agents such as benzyl alcohol, antioxidants such as ascorbic acid or sodium bisulphite, chelating agents such as ethylene diamine-tetra-acetic acid, buffers such as acetates, citrates or phosphates and agents for adjusting the osmolarity, such as sodium chloride or dextrose.

These pharmaceutical forms are prepared using methods which are routinely used by pharmacists.

The percentage of active material in the pharmaceutical compositions can fall within a wide range of concentrations and depends on a variety of factors such as the patient's sex, age, weight and medical condition, as well as on the method of administration. Thus the quantity of active product in compositions for oral administration is at least 0.5% by weight and can be up to 80% by weight with respect to the composition weight.

In compositions for parenteral administration, the quantity of active material present is at least 0.5% by weight and can be up to 33% by weight with respect to the composition weight. For the preferred parenteral compositions, the dosage unit is in the range 0.5 mg to 5.000 mg of active product.

The daily dose can fall within a wide range of dosage units of active product, and is generally in the range of 0.01 to 100 mg/kilogram (kg). However, it should be understood that the specific doses can be adapted to particular cases depending on the individual requirements, at the physician's discretion.

The present invention concerns also a pharmaceutical composition comprising (S)-(-)-α-ethyl-2-oxo-1-pyrrolidineacetamide or ((2-S) -2-[(4S)-4*-*(2,2-diflurovinyl)-2-oxopyrrolidinyl] butanamide and at least one compound having anti-parkinsonian least one compound having anti-parkinsonian activity, selected from amantadine, and ropinirole.

The present invention relates to a method of selectively potentiating the therapeutic effect of the compound having anti-parkinsonian activity L-DOPA without increasing undesired side effects associated therewith which comprises co-administration of an amount of the said compound with an amount of (S)-(-)-α-ethyl-2-oxo-1-pyrrolidineacetamide effective in producing the desired therapeutic effect.

### Example 1

This study was designed to investigate whether levetiracetam has anti-dyskinetic activity in the 1-methyl-4-phenyl-1,2,3,6-tetrahydropyridine (MPTP) - lesioned marmoset model of Parkinson's disease. The effect of levetiracetam on L-3.4-dihydroxyphenylalanine (L-DOPA)-induced dyskinesias and alleviation of parkinsonism symptoms was investigated.

The study was performed on seven adult marmosets (*Callithrix jacchus)* bred in a closed colony. The marmosets were rendered parkinsonian by subcutaneous injection of 2mg/kg MPTP for 5 consecutive days. The marmosets were allowed to recover for 18 weeks until their parkinsonism became stable. The degree of activity and disability before and after MPTP treatment was assessed using a combination of scales that measure locomotor activity, mobility, bradykinesia and posture. Animals were treated with L-DOPA (12.5 mg/kg b.i.d. for 6 weeks) to prime them to elicit dyskinesia. After this time all animals demonstrated stable levels of dyskinesia when challenged with L-DOPA.

All drugs were administered orally in a volume of 5 ml/kg via a syringe in the animal's home cage. The animals were immediately transferred to the experimental cage (60cm x 55cm x 75cm, with the perch 25cm from floor of cage) for behavioural assessment. Vehicle was apple juice in all cases.

A battery of behavioural tests were performed:
1) Activity - a quantitative assessment of the amount of movement of the animal was obtained every 5 minutes for the duration of the experiment using computer-based activity monitors.
2) Parkinsonian disability - non-parametric measures based on the following scales
   a) Range of movement score: 0 = no movement. 1= movement of head on the floor of the cage, 2 = movement of limbs, but no locomotion, on the floor of the cage, 3 = movement of head or trunk on wall of cage or perch. 4 = movement of limbs, but no locomotion, on wall of cage or perch, 5 = walking around floor of cage or eating from hopper on floor, 6 = hopping on floor of cage, 7 = climbing onto wall of cage or perch, 8 = climbing up and down the walls of the cage or along perch, 9 = running, jumping, climbing between cage walls / perch / roof, uses limbs through a wide range of motion and activity.
   b) Bradykinesia score: 0 = normal speed and initiation of movement, 1 = mild slowing of movement, 2 = moderate slowing, difficulty initiating and maintaining movement, marked freezing, 3 = akinetic, unable to move, with prolonged freezing episodes
   c) Postural abnormality score: 0 = normal, upright, holds head up, normal balance, 1 = abnormal, crouched, face down, may lose balance.
   d) Parkinsonian disability score: A combination of the mobility, bradykinesia and posture scores according to the formula (18- (Range of movement *2) + (Bradykinesia * 3) + (posture * 9)] to give a global parkinsonian disability rating.
3)Dyskinesia - non-parametric measures based on the following scale
   Dyskinesia score: 0 = Absent, 1 = Mild, fleeting, 2 = Moderate, not interfering with normal activity, 3 = Marked, at times interfering with normal activity, 4 = Severe, continuous, replacing normal activity.

Behaviour was assessed for 6 hours post drug administration. Behavioural test 1, activity, was assessed every 5 minutes for 6 hours post drug administration. Behavioural tests 2 and 3, parkinsonian disability and dyskinesia, respectively, were assessed for 10 minutes every 30 minutes over the course of 6 hours, by post hoc analysis of video-recordings by an observer blinded to the treatment. The score given in each 10 minutes time period represents the maximum score achieved during that time period.

Table 1 outlines the randomised treatment schedule i.e. three doses of levetiracetam drug in combination with a single dose of L-DOPA. The actions of each of these three combination therapies were compared with that of L-DOPA plus the appropriate vehicle. Thus, a total of four treatments were given.

**Table 1 - Randomised treatment schedule**

| Date / animal number | | | | | | | |
|---|---|---|---|---|---|---|---|
| | 1 | 2 | 3 | 4 | 5 | 6 | 7 |
| Day 1 | V | D1 | D2 | D1 | D2 | D3 | V |
| Day 4 | D1 | D3 | V | D3 | V | D2 | D3 |
| Day 6 | D3 | D2 | D1 | D2 | D1 | V | D1 |
| Day 8 | D2 | V | D3 | V | D3 | D1 | D2 |

| | | | | | | | |
|---|---|---|---|---|---|---|---|
| V = L-DOPA + vehicle D1= L-DOPA (12mg/kg) + levetiracetam (13mg/kg) D2 = L-DOPA (12mg/kg) + levetiracetam (30mg/kg) D3 = L-DOPA (12mg/kg) + levetiracetam (60mg/kg) L-DOPA (12mg/kg) alone reversed parkinsonian symptoms. The alleviation of parkinsonian symptoms was accompanied by severe dyskinesia. | | | | | | | |

Dyskinesia was significantly reduced following the combined treatment for the first hour post drug administration (p < 0.01, p < 0.05 and p < 0.01 for 13mg/kg, 30mg/kg and 60mg/kg, respectively; Friedman test followed by Dunn's multiple comparison's test). In contrast, there were no significant differences in disability scores for the first hour post drug administration (p > 0.05 for 13mg/kg, 30mg/kg and 60mg/kg; Friedman test followed by Dunn's multiple comparison's test). Co-administration of levetiracetam (13 to 60 mg/kg) and L-DOPA (12mg/kg) reversed parkinsonian symptoms to the same magnitude, at peak effect, as L-DOPA (12mg/kg) monotherapy. There were no significant differences in dyskinesia or disability scores at any time-point after one hour post drug administration (p > 0.05 for 13mg/kg, 30mg/kg and 60mg/kg; Friedman test followed by Dunn's multiple comparison's test).

Combined levetiracetam (13-60mg/kg) and L-DOPA (12mg/kg) treatment had the same maximal anti-parkinsonian action compared to L-DOPA monotherapy.

Combined levetiracetam (13-60mg/kg) and L-DOPA (12mg/kg) treatment was associated with less significantly dyskinesia, during the first hour post drug administration, than L-DOPA monotherapy.

In combination with L-DOPA, levetiracetam had a significant advantage over L-DOPA monotherapy.

The major benefit of levetiracetam was a reduction in L-DOPA-induced dyskinesia during the first hour post drug administration. This reduction in dyskinesia was seen without a reduction in anti-parkinsonian efficacy.

Thus, the clinical beneficit for levetiracetam may be as an adjunctive therapy to reduce dyskinesia in parkinson patients exposed to chronic dopamine replacement therapy using L-DOPA.

### Example 2

This study was designed to investigate whether the compound ((2S)-2-[(4S)-4-(2,2-difluorovinyl)-2-oxopyrrolldinyl]butanamide) (named compound A) has anti-dyskinetic activity in the 1-methyl-4-phenyl-1,2,3,6-tetrahydropyridine (MPTP) - lesioned marmoset model of Parkinson's disease. The effect of the compound A on L-3,4-dihydroxyphenylalanine (L- DOPA)-induced dyskinesias and alleviation of anti parkinsonism symptoms was investigated.

The study was performed on nine adult marmosets (*Callithrix jacchus*) bred in a closed colony. The marmosets were rendered parkinsonian by subcutaneous injection of 2mg/kg MPTP for 5 consecutive days. The marmosets were allowed to recover for 18 weeks until their parkinsonism became stable. The degree of activity and disability before and after MPTP treatment was assessed using a combination of scales that measure locomotor activity, mobility, bradykinesia and posture. Animals were treated with L-DOPA (13.9+/-0.8mg/kg b.i.d. for 6 weeks) to prime them to elicit dyskinesia. After this time all animals demonstrated stable levels of dyskinesia when challenged with L-DOPA .

All drugs were administered orally in a volume of 5 ml/kg via a syringe in the animal's home cage. The animals were immediately transferred to the experimental cage (60cm x 55cm x 75cm, with the perch 25cm from floor of cage) for behavioural assessment. Vehicle was apple juice in all cases.

A battery of behavioural tests were performed:
1) Activity - a quantitative assessment of the amount of movement of the animal was obtained every 5 minutes for the duration of the experiment using computer-based activity monitors.
2) Parkinsonian disability - non-parametric measures based on the following scales
   a) Range of movement score: 0 = no movement, 1= movement of head on the floor of the cage, 2 = movement of limbs, but no locomotion, on the floor of the cage, 3 = movement of head or trunk on wall of cage or perch, 4 = movement of limbs, but no locomotion, on wall of cage or perch, 5 = walking around floor of cage or eating from hopper on floor, 6 = hopping on floor of cage, 7 = climbing onto wall of cage or perch, 8 = climbing up and down the walls of the cage or along perch, 9 = running, jumping, climbing between cage walls / perch / roof, uses limbs through a wide range of motion and activity.
   b) Bradykinesia score: 0 = normal speed and initiation of movement, 1 = mild slowing of movement, 2 = moderate slowing, difficulty initiating and maintaining movement, marked freezing, 3 = akinetic, unable to move, with prolonged freezing episodes
   c) Postural abnormality score: 0 = normal, upright, holds head up, normal balance.1 = abnormal, crouched, face down, may lose balance.
   d) Parkinsonian disability score: A combination of the mobility, bradykinesia and posture scores according to the formula [18 - (Range of movement * 2) + (Bradykinesia *3) + (posture *9)] to give a global parkinsonian disability rating.
3)Dyskinesia - non-parametric measures based on the following scale
   Dyskinesia score: 0 = Absent, 1 = Mild, fleeting, 2 = Moderate, not interfering with normal activity, 3 = Marked, at times interfering with normal activity, 4 = Severe, continuous, replacing normal activity.

Behaviour was assessed for 6 hours post drug administration. Behavioural test 1, activity, was assessed every 5 minutes for 6 hours post drug administration. Behavioural tests 2 and 3, parkinsonian disability and dyskinesia, respectively, were assessed for 10 minutes every 30 minutes over the course of 6 hours, by *post hoc* analysis of video-recordings by an observer blinded to the treatment. The score given in each 10 minutes time period represents the maximum score achieved during that time period.

Four doses of compound A drug (1mg/kg . 3mg/kg . 10mg/kg and 30mg/kg) in combination with a single dose of L-DOPA were tested using a randomized treatment schedule. The actions of each of these four combination therapies were compared with that of L-DOPA plus the appropriate vehicle. Thus, a total of five treatments were given.

L-DOPA alone reversed parkinsonian symptoms. The alleviation of parkinsonian symptoms was accompanied by dyskinesia.

At the doses of 10mg/kg and 30mg/kg of compound A, dyskinesia was significantly reduced following the combined treatment with L-DOPA for the first hour post drug administration ( p>0.05 for 1mg/kg and 3mg/kg , p<0.05 for 10mg/kg and 30mg/kg ; Friedman test followed by Dunn's multiple comparison's test). In contrast, there were no significant differences in disability scores for the first hour post drug administration (p > 0.05 for 1mg/kg, 3mg/kg , 10mg/kg and 30mg/kg; Friedman test followed by Dunn's multiple comparison's test). Co-administration of compound A (1 to 30 mg/kg) and L-DOPA (13.9+/-0.8mg/kg) reversed parkinsonian symptoms to the same magnitude, at peak effect, as L-DOPA (13.9+/-0.8mg/kg) monotherapy. There were no significant differences in dyskinesia or disability scores at any time-point after one hour post drug administration (p > 0.05 for 1mg/kg, 3mg/kg , 10mg/kg and 30mg/kg; Friedman test followed by Dunn's multiple comparison's test).

Combined compound A (1-30mg/kg) and L-DOPA (13.9+/-0.8mg/lcg) treatment had the same maximal anti-parkinsonian action compared to L-DOPA monotherapy.

Combined compound A (10 and 30mg/kg) and L-DOPA (13.9+/-0.8mg/kg) treatment was associated with less significantly dyskinesia, during the first hour post drug administration, than L-DOPA monotherapy.

In combination with L-DOPA, compound A had a significant advantage over L-DOPA monotherapy.

The major benefit of compound A was a reduction in L-DOPA-induced dyskinesia during the first hour post drug administration. This reduction in dyskinesia was seen without a reduction in anti-parkinsonian efficacy.

Thus, the clinical beneficit for compound A may be as an adjunctive therapy to reduce dyskinesia in parkinson patients exposed to chronic dopamine replacement therapy, using L-DOPA.

### Example 3

This study was designed to investigate whether Levetiracetam has a potential as an adjunctive anti-parkinsonian agent to dopamine replacement therapy in the 1-methyl-4-phenyl-1,2,3,6-tetrahydropyridine (MPTP)-lesioned marmoset model of Parkinson's disease. The effect of Levetiracetam on Ropinirole alleviation of parkinsonism symptoms was investigated.

The study was performed on six adult marmosets (*Callithrix jacchus* ; 4 female, 2 male). The marmosets were rendered parkinsonian by subcutaneous injection of 2mg/kg MPTP for 5 consecutive days. The marmosets were allowed to recover for 18 weeks until their parkinsonism was stable. The degree of activity and disability before and after MPTP treatment were assessed using a combination of scales that measure locomotor activity, mobility, bradykinesia and posture. Animals were treated with L-DOPA 12mg/kg b.i.d. for 6 weeks. After this time, animals were used for assessment of potential symptomatic antiparkinsonian therapy. All drugs were administered orally in a volume of 5 ml/kg via a syringe in the animal's home cage. The animals were immediately transferred to an experimental cage (60cm x 55cm x 75cm, with the perch 25cm from floor of cage) for behavioural assessment. Vehicle was apple juice in all cases. The doses were 3.75 mg/kg of Ropinirole in combination with Levetiracetam at 13. 30 and 60 mg/kg. Behaviour was assessed for 6 hours post drug administration.

A battery of behavioural tests was performed:
1) Activity -a quantitative assessment using computer-based activity monitors was obtained every 5 minutes for the duration of the experiment.
2) Parkinsonian disability -non-parametric measures based on the following scales:
   a) Range of movement score: 0 = no movement, 1 = movement of head on the floor of the cage, 2 = movement of limbs, but no locomotion, on the floor of the cage, 3 = movement of head or trunk on wall of cage or perch, 4 = movement of limbs, but no locomotion, on wall of cage or perch, 5 = walking around floor of cage or eating from hopper on floor, 6 = hopping on floor of cage, 7 = climbing onto wall of cage or perch, 8 = climbing up and down the walls of the cage or along perch, 9 = running, jumping, climbing between cage walls / perch / roof, uses limbs through a wide range of motion and activity. The score given was the maximum achieved in each 10 minute observation period.
   b) Bradykinesia score: 0 = normal speed and initiation of movement, 1 = mild slowing of movement, 2 = moderate slowing, difficulty initiating and maintaining movement, marked freezing. 3 = akinetic, unable to move, with prolonged freezing episodes. The score given was representative of behaviour over the observation period.
   c) Postural abnormality score: 0 = normal, upright, holds head up, normal balance, 1 = abnormal, crouched, face down, may lose balance. The score given was representative of behaviour over the observation period.
   d) Parkinsonian disability score: A combination of the mobility, bradykinesia and posture scores according to the formula [18 - (Range of movement * 2) + (Bradykinesia * 3) + (Posture * 9)] to give a global parkinsonian disability rating.

Behavioural test 1 (activity) was assessed every 5 minutes for 6 hours post drug administration. Behavioural tests 2 (parkinsonian disability) was assessed for 10 minutes every 30 minutes over the course of 6 hours, by *post hoc* analysis of video-recordings by an observer blinded to the treatment. The score given / achieved in each 10 minute time period was presented.
Range of movement score: 0 = none, 3 = low, 6 = moderate. 9 = high
Bradykinesia score: 0 = none, 1 = mild, 2 = moderate, 3 = severe
Postural abnormality score: 0 = none, 0.5 = mild, 1 = severe
Parkinsonian disability score: 0 = none, 9 = mild, 18 = moderate. 27 = marked, 36 = severe

Cumulated data for parkinsonian disability, range of movement, bradykinesia and postural abnormalities were analysed with a non-parametric repeated measures one-way ANOVA (Friedman's test) followed by Dunn's multiple comparison test (Graphpad Prism version 3).

Levetiracetam, administered at 13 and 30mg/kg but not 60 mg/kg, significantly potentiated the alleviation of parkinsonism by Ropinirole (3.75mg/kg). Thus, Levetiracetam, administered at 13 and 30mg/kg significantly increased activity and "on-time" (all P < 0.01; one-way repeated measures ANOVA followed by Dunnett's multiple comparison's test). Also, Levetiracetam administered at 13mg/kg significantly reduced parkinsonian disability over the experiment as a whole and specifically during 3-4 hour time period (P < 0.05; Friedman's test followed by Dunn's multiple comparison's test). Furthermore, Levetiracetam, administered at 30mg/kg, significantly increased range of movement during the 0-1 hour time period (P < 0.05; Friedman's test followed by Dunn's multiple comparison's test). In conclusion , the increase in general activity levels was accompanied by a significant reduction in parkinsonian disability and reflects an enhancement of the anti-parkinsonian actions afforded by Ropinirole. Furthermore, there was an enhancement of "on-time" by approximately 82% and 69% for 13mg/kg and 30mg/kg Levetiracetam, respectively. However, activity counts were still elevated at the end of the six hour experiment suggesting that observed "on-time" might have been greater if the experiment had not been terminated at six hours.

Levetiracetam may have potential as an anti-parkinsonian agent in combination with dopamine replacement therapy. The extension of "on-time" might represent a useful *de novo* therapy to delay the onset of dyskinesia.

## Claims

1. Use of a 2-oxo-1-pyrrolidine derivative selected from (S)-(-)-α-ethyl-2-oxo-1-pyrrolidine-acetamide and ((2S)-2-[(4S)-4-(2,2-difluorovinyl)-2-oxopyrrolidinyl]butanamide) for the manufacture of a medicament for the treatment of Parkinson's Disease in a chronic dopamine replacement therapy using L-DOPA.

2. Use of a 2-oxo-1-pyrrolidine derivative selected from (S)-(-)-α-ethyl-2-oxo-1-pyrrolidine-acetamide and ((2S)-2-[(4S)-4-(2,2-difluorovinyl)-2-oxopyrrolidinyl]butanamide) in combination with either amantadine or ropinirole for the manufacture of a medicament for the treatment of Parkinson's Disease in a dopamine replacement therapy using L-DOPA.

3. A pharmaceutical composition comprising (S)-(-)-α-ethyl-2-oxo-1-pyrrolidine-acetamide) and amantadine as well as a pharmaceutically acceptable carrier.

4. A pharmaceutical composition comprising (S)-(-)-a-ethyl-2-oxo-1-pyrrolidine-acetamide and ropinirole as well as a pharmaceutically acceptable carrier.

5. A pharmaceutical composition comprising ((2S)-2-[(4S)-4-(2,2-difluorovinyl)-2-oxopyrrolidinyl]butanamide and amantadine as well as a pharmaceutically acceptable carrier.

6. A pharmaceutical composition comprising ((2S)-2-[(4S)-4-(2,2-difluorovinyl)-2-oxopyrrolidinyl]butanamide and ropinirole as well as a pharmaceutically acceptable carrier.

## Patentansprüche

1. Verwendung eines 2-Oxo-1-pyrrolidin-Derivats, ausgewählt aus (S)-(-)-α-Ethyl-2-oxo-1-pyrrolidinacetamid und ((2S)-2- [(4S)-4-(2,2-Difluorvinyl)-2-oxopyrrolidinyl] butanamid), zur Herstellung eines Medikaments für die Behandlung von Parkinson-Krankheit bei einer chronischen Dopamin-Substitutionstherapie unter Verwendung von L-DOPA.

2. Verwendung eines 2-Oxo-1-pyrrolidin-Derivats, ausgewählt aus (S)-(-)-α-Ethyl-2-oxo-1-pyrrolidinacetamid und ((2S)-2-[(4S)-4-(2,2-Difluorvinyl)-2-oxopyrrolidinyl]butanamid), in Kombination mit entweder Amantadin oder Ropinirol zur Herstellung eines Medikaments für die Behandlung von Parkinson-Krankheit bei einer Dopamin-Substitutionstherapie unter Verwendung von L-DOPA.

3. Pharmazeutische Zusammensetzung, umfassend (S)-(-)-α-Ethyl-2-oxo-1-pyrrolidin-acetamid und Amantadin sowie einen pharmazeutisch akzeptablen Träger.

4. Pharmazeutische Zusammensetzung, umfassend (S)-(-)-α-Ethyl-2-oxo-1-pyrrolidin-acetamid und Ropinirol sowie einen pharmazeutisch akzeptablen Träger.

5. Pharmazeutische Zusammensetzung, umfassend ((2S)-2-[(4S)-4-(2,2-Difluorvinyl)-2-oxo-pyrrolidinyl]butanamid) und Amantadin sowie einen pharmazeutisch akzeptablen Träger.

6. Pharmazeutische Zusammensetzung, umfassend ((2S)-2-[(4S)-4-(2,2-Difluorvinyl)-2-oxo-pyrrolidinyl]butanamid) und Ropinirol sowie einen pharmazeutisch akzeptablen Träger.

## Revendications

1. Utilisation d'un dérivé de la 2-oxo-1-pyrrolidine choisi parmi le (S)-(-)-α-éthyl-2-oxo-1-pyrrolidine-acétamide et le ((2S)-2-[(4S)-4)-2,2-difluorovinyl)-2-oxopyrrolidinyl] butanamide) pour la fabrication d'un médicament de traitement de la maladie de Parkinson dans une thérapeutique de remplacement chronique de la dopamine utilisant L-DOPA.

2. Utilisation d'un dérivé de la 2-oxo-1-pyrrolidine choisi parmi le (S)-(-)-α-éthyl-2-oxo-1-pyrrolidine-acétamide et le ((2S)-2-[(4S)-4)-2,2-difluorovinyl)-2-oxopyrrolidinyl]butanamide) en combinaison avec de l'amantadine ou du ropinirole pour la fabrication d'un médicament de traitement de la maladie de Parkinson dans une thérapeutique de remplacement de la dopamine utilisant L-DOPA.

3. Composition pharmaceutique comprenant du (S)-(-)-α-éthyl-2-oxo-1-pyrrolidine-acétamide et de l'amantadine, ainsi qu'un véhicule acceptable pharmaceutiquement.

4. Composition pharmaceutique comprenant du (S)-(-)-α-éthyl-2-oxo-1-pyrrolidine-acétamide et du ropinirole, ainsi qu'un véhicule acceptable pharmaceutiquement.

5. Composition pharmaceutique comprenant du ((2S)-2-[(4S)-4)-2,2-difluorovinyl)-2-oxopyrrolidinyl]butanamide) et de l'amantadine ainsi qu'un véhicule acceptable pharmaceutiquement.

6. Composition pharmaceutique comprenant du ((2S)-2-[(4S)-4)-2,2-difluorovinyl)-2-oxopyrrolidinyl]butanamide) et du ropinirole, ainsi qu'un véhicule acceptable pharmaceutiquement.
